# EUROPÄISCHE PATENTSCHRIFT

(11) **EP 1 288 180 B1**
(45) Veröffentlichungstag und Bekanntmachung des Hinweises auf die Patenterteilung: **02.11.2005**
(21) Anmeldenummer: 02018153.3
(22) Anmeldetag: 19.08.2002
(51) Int. Cl.: C07C 6/06

(54) **Verfahren zur Herstellung von Cycloalkadienen durch Metathesereaktion**
Process to prepare cycloalkadienes via a metathesis reaction
Procédé pour préparer par métathèse des cycloalcadiènes

(30) Priorität: 28.08.2001 DE 10142032
(43) Veröffentlichungstag der Anmeldung: 05.03.2003
(73) Patentinhaber: Symrise GmbH & Co. KG, 37603 Holzminden (DE)
(72) Erfinder: Wöhrle, Ingo, Dr., 37603 Holzminden (DE); Esser, Peter, Dr., Summerville, SC 29485 (US); Reckziegel, Aurélia, Dr., 37603 Holzminden (DE); Brandt, Matthias, Dr., 40589 Düsseldorf (DE); Klein, Stephan, Dr., 51467 Bergisch Gladbach (DE); Turek, Thomas, Dr., 40239 Düsseldorf (DE)
(74) Vertreter: Eisenführ, Speiser & Partner

(56) Entgegenhaltungen:
- DE-A- 19 727 256
- GB-A- 1 118 517
- US-A- 4 668 836

## Beschreibung

Die Erfindung betrifft ein Verfahren zur Herstellung von Cycloalkadienen mit Trägerkatalysatoren auf Basis von Re₂O₇/γ-Al₂O₃.

Cycloalkene, insbesondere Cycloalkadiene mit einer Ringgröße zwischen 12 und 18 Kohlenstoffatomen, werden unter anderem zur Herstellung von sauerstoffhaltigen, makrocyclischen Verbindungen verwendet. Die Verbindungen können eingesetzt werden bei der Herstellung makrocyclischer Ketone, Lactone und Epoxide, die in der Duftstoffindustrie als Moschusriechstoffe begehrt sind.

Aus der EP-A 182 333 ist bekannt, dass bei Verwendung des Katalysatorsystems Re₂O₇ / γ-Al₂O₃ / SnR₄, wobei R für einen Alkylrest steht, durch Metathesereaktion hochverdünnter Cycloolefinlösungen in Flüssigphase die entsprechenden Cycloalkadiene erhalten werden können.

Die Herstellung von Cycloalkadienen in Flüssigphase in Gegenwart eines Trägerkatalysators auf Basis Re₂O₇ / γ-Al₂O₃ durch Metathesereaktion von Cyclooctenylenen mit einem Polymerisierungsgrad größer oder gleich drei und / oder Cycloalkamonoenen ist in EP-A 343 437 beschrieben.

In der Chemiker-Zeitung 1983, 107, 115 ist die Herstellung von Cycloalkadienen am Re₂O₇ / γ-Al₂O₃ - Kontakt beschrieben. Als Trägermaterial wurde γ-Al₂O₃-CK-300 der Firma Akzo verwendet.

In EP-B 991 467 sind Boroxid-haltige Re₂O₇ / γ-Al₂O₃ - Katalysatoren in Form von Strangpresslingen beschrieben.

Aufgrund der notwendigen hohen Verdünnung der in die Metathesereaktion eingesetzten Cycloolefinlösungen, ist die pro Zeiteinheit erhältliche Menge an Cycloalkadienen bisher unter wirtschaftlichen, technischen sowie industriellen Aspekten unbefriedigend.

Es bestand daher die Aufgabe Trägerkatalysatoren und Verfahren bereitzustellen, mittels derer eine größere Menge an Cycloalkadienen pro Zeiteinheit erhalten werden kann. Es ist von großem Vorteil, eine höhere Produktivität und eine höhere Raum-Zeit-Leistung in dem Metatheseverfahren zu erzielen.

Überraschenderweise wurde nun gefunden, dass Trägerkatalysatoren mit hoher spezifischer äußerer Oberfläche eine deutliche Zunahme der Aktivität und Produktivität des Re₂O₇ / γ-Al₂O₃ - Trägerkatalysators bewirken können. Dies macht sich insbesondere bei höheren Raum-Zeit-Geschwindigkeiten bemerkbar, wodurch die pro Zeiteinheit erhältliche Menge an Cycloalkadienen deutlich gesteigert werden kann. Weiterhin wurde gefunden, dass innerhalb eines Trägerkatalysatorzyklus mehr Metatheseprodukte und Cycloalkadiene hergestellt werden können.

Die Erfindung betrifft daher ein Verfahren zur Herstellung von Cycloalkadienen aus Cycloalkamonoenen, Cyclopolyenen, acyclischen Polyenen oder deren Gemische in Flüssigphase durch Metathesereaktion in Gegenwart eines Trägerkatalysator-Formkörpers auf Basis Re₂O₇ / γ-Al₂O₃, dadurch gekennzeichnet, dass die berechnete spezifische äußere Oberfläche des Trägerkatalysator-Formkörpers größer oder gleich 3,5 mm² / mm³ ist.

Unter Metathese-Lösung wird in der vorliegenden Erfindung die Edukt-Lösung, d.h. ein Lösungsmittel enthaltend mindestens einen Kohlenwasserstoff aus der Reihe der Cycloalkamonoene, Cyclopolyene oder acyclischen Polyene, verstanden.

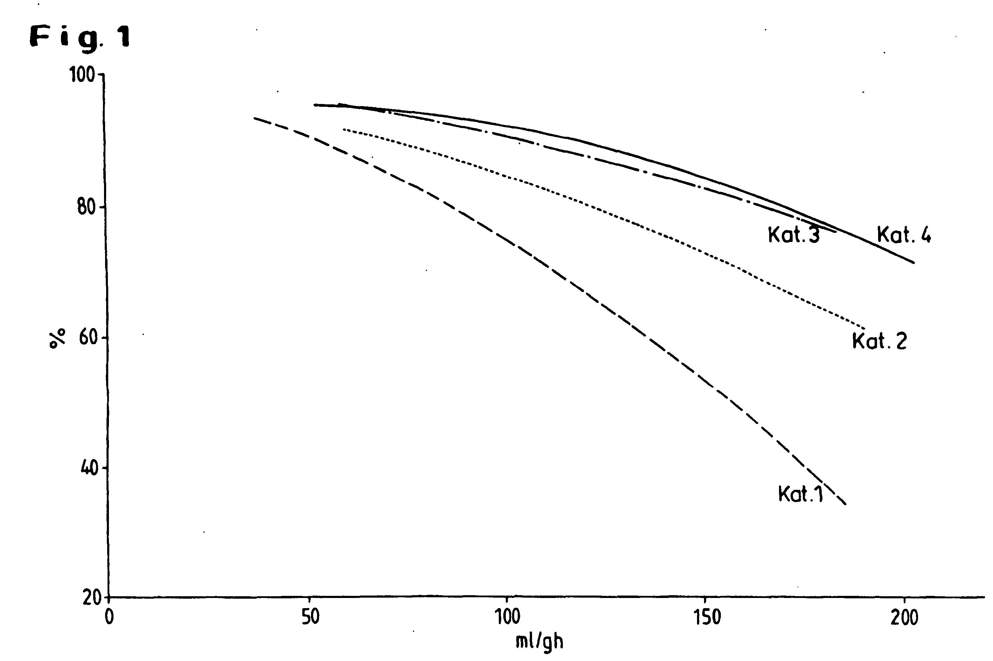

In Fig. 1 ist der Cycloocten-bezogene Umsatz (in Prozent, x-Achse) in Abhängigkeit von der Raum-Zeit-Geschwindigkeit (in ml / gh, y-Achse) in einer Metathesereaktion mit den erfindungsgemäßen Trägerkatalysatoren (Kat. 2 bis 4) im Vergleich zu einem kommerziell erhältlichen Trägerkatalysator Kat. 1 graphisch dargestellt.

Die erfindungsgemäßen Trägerkatalysatoren zeigen eine deutlich höhere Aktivität, wodurch eine höhere Raum-Zeit-Leistung und eine höhere Produktivität an Cycloalkadienen erreicht werden kann. Die Trägerkatalysatoren sind in Tabelle 2 und Beispiel 1, die Versuchsbedingungen in Beispiel 2 näher dargelegt.

Unter der spezifischen äußeren Oberfläche wird das Verhältnis aus berechneter, geometrischer äußerer Oberfläche und dem berechneten äußeren Gesamtvolumen des Trägerkatalysator-Formkörpers verstanden.

Geeignete Trägerkatalysatoren haben eine spezifische äußere Oberfläche von größer oder gleich 3,5 mm²/mm³, bevorzugt eine von größer oder gleich 4,0 mm²/mm³, besonders bevorzugt eine von größer oder gleich 5,0 mm²/mm³.

Die Trägerkatalysatoren werden als Formkörper jeglicher Gestalt wie beispielsweise Hohlsträngen, Extrudaten, Strangpresslingen, Kugeln, Hohlzylindern, Zylindern, Würfeln, Kegeln und dergleichen eingesetzt. Besonders bevorzugt sind Kugeln, Wirbelstränge (WS) oder Zylinder.

Die Schüttdichte der Trägerkatalysatoren liegt typischerweise im Bereich 400 bis 900 g/l.

Die Trägerkatalysatoren weisen typischerweise spezifische Oberflächen von 100 bis 300 m²/g nach BET (Bestimmungsmethode nach Brunauer, Emmett und Teller) auf.

Zur Verdeutlichung der Ermittlung der spezifischen äußeren Oberfläche sind in Tabelle 1 einige nicht limitierende Beispiele genannt.

In Tabelle 1 verwendete Abkürzungen und Symbole: 0: Durchmesser, Volumen: äußeres Gesamtvolumen; Ober.: äußere Oberfläche; spez. Ober: spezifische äußere Oberfläche.

**Tabelle 1:**

| Berechnung der spezifischen äußeren Oberfläche | | | | | |
|---|---|---|---|---|---|
| | | Kugel | Kugel | Zylinder | Hohlzylinder |
| ∅ außen | mm | 1,0 | 1,5 | 0,8 | 3,5 |
| ∅ innen | mm | | | | 2 |
| Länge | mm | | | 6 | 4 |
| Volumen | mm³ | 0,52 | 1,77 | 3,02 | 38,5 |
| Ober. | mm² | 3,14 | 7,07 | 16,09 | 82,1 |
| spez. Ober. | mm² / mm³ | 6,00 | 4,00 | 5,33 | 2,13 |

Bevorzugt ist eine kontinuierliche Reaktionsführung, insbesondere eine vertikale Anordnung der Trägerkatalysatoren in einem Festbett, wobei vorteilhafterweise das Festbett von unten nach oben von der Metathese-Lösung durchströmt wird.

Der Gehalt an Re₂O₇ auf dem Trägerkatalysator, bezogen auf das Trägerkatalysatorgewicht, liegt vorteilhafterweise im Bereich von 1 bis 12 Gew.%, bevorzugt im Bereich von 2 bis 8 Gew.%, insbesondere im Bereich 3 bis 6 Gew.%. Die Herstellung der Trägerkatalysatoren erfolgt mit dem Fachmann bekannten Verfahrensweisen. Das Aufbringen des Rheniums erfolgt üblicherweise durch Imprägnierung oder Tränken des Trägermaterials mit einer wässrigen Lösung einer oder mehrerer Rheniumverbindungen und anschließender thermischer Behandlung des Guts, wobei Re₂O₇ entsteht. Geeignete Rheniumverbindungen sind beispielsweise Perrhenate wie z.B. Ammoniumperrhenat, es kann auch Perrheniumsäure oder Rheniumheptoxid selbst verwendet werden. Die thermische Behandlung des Trägerkatalysators erfolgt in einem Temperaturbereich von 200 bis 600°C, wobei eine Maximaltemperatur im Bereich von etwa 600°C einzuhalten ist.

Es ist besonders vorteilhaft, wenn der Trägerkatalysator 0,5 bis 40 Gew.%, bevorzugt 1 bis 20 Gew.%, insbesondere 1 bis 10 Gew.% eines Zinntetraalkyls oder Zinndioxid oder eines Gemisches dieser Zinnverbindungen enthält. Als bevorzugte Zinntetraalkyle seien Zinntetramethyl, Zinntetraethyl, Zinntetra-n-butyl, Zinntetra-n-octyl genannt, insbesondere bevorzugt ist Zinntetramethyl. Es ist besonders vorteilhaft, wenn der Trägerkatalysator vor Beginn der Metathesereaktion mit einer Zinntetraalkyl-haltigen Lösung in Kontakt gebracht wird, wobei auch Mischungen der genannten Zinntetraalkyle eingesetzt werden können. Die Belegung mit Zinndioxid kann beispielsweise bei der Regenerierung des Zinntetraalkyl-haltigen Trägerkatalysators erfolgen, aber auch dadurch erreicht werden, dass der Trägerkatalysator mit wasserlöslichen Zinnverbindungen imprägniert oder getränkt und anschließend in sauerstoffhaltiger Atmosphäre auf 500 - 600°C erhitzt wird, wobei Zinnoxid entsteht.

Weiterhin ist es vorteilhaft, wenn die Metathesereaktion in Gegenwart eines Zinntetraalkyls durchgeführt wird. Typischerweise werden die Zinntetraalkyle vor Beginn der Metathesereaktion in die Metathese-Lösung zugegeben, und dieses Gemisch aus einem Vorratsbehältnis über das Trägerkatalysatorbett geführt. Die Zinntetraalkyle werden typischerweise in einer Menge von 0,1 bis 8 Gew.%, bevorzugt 0,1 bis 5 Gew.%, insbesondere 0,1 bis 2,5 Gew.%, bezogen auf das Trägerkatalysatorgewicht, der Metathese-Lösung zugesetzt. Bevorzugte Zinntetraalkyle sind Zinntetramethyl, Zinntetraethyl, Zinntetra-n-butyl, Zinntetra-n-octyl, insbesondere bevorzugt ist Zinntetramethyl.

Vorteilhaft ist die Behandlung des Trägerkatalysators mit einer oder mehreren Mineralsäuren, wobei die Behandlung vor oder nach der Aufbringung des Rheniums erfolgen kann. Bevorzugt ist die Behandlung des γ-Al₂O₃-Trägermaterials oder des Rebeladenen Trägerkatalysators mit einer wässrigen HCl-Lösung.

Weiterhin vorteilhaft sind Trägerkatalysatoren, die 0,2 bis 3 Gew.% an Cäsium enthalten, wobei die Behandlung mit einer oder mehreren Cäsiumverbindungen vor oder nach der Aufbringung des Rheniums erfolgen kann. Bevorzugt ist die Behandlung mit einer wässrigen Cäsiumnitrat-Lösung.

Weiterhin vorteilhaft sind Trägerkatalysatoren, die 0,3 bis 3 Gew.% an Phosphor enthalten, wobei die Behandlung mit einer oder mehreren Phosphorverbindungen vor oder nach der Aufbringung des Rheniums erfolgen kann. Bevorzugt ist die Behandlung mit einer wässrigen Ammoniumphosphat-Lösung, besonders bevorzugt mit Diammoniumhydrogenphosphat.

Die genannten Dotierungen, Belegungen oder Behandlungen werden bevorzugt mittels Imprägnierung oder Tränkung auf den Trägerkatalysator aufgebracht, die Herstellung der Trägerkatalysatoren mittels Digerieren ist auch möglich.

Der Gehalt in der Flüssigphase an Cycloalkamonoenen, Cyclopolyenen, acyclischen Polyenen oder deren Gemische liegt typischerweise im Bereich von 0,5 bis 10 g/l, bevorzugt im Bereich 1,0 bis 5,5 g/l, insbesondere im Bereich 2,0 bis 4,0 g/l.

Die Edukte werden in Metathese-inerten Lösungsmitteln eingesetzt. Geeignete Lösungsmittel sind beispielsweise Kohlenwasserstoffe und halogenierte Kohlenwasserstoffe, insbesondere Butan, Pentan, Hexan, Heptan, Octan, Cyclopentan, Cyclohexan, Cyclooctan, Dichlormethan und Trichlorethan. Bevorzugt sind n-Pentan, n-Hexan, n-Heptan, n-Octan, iso-Octan, Cyclopentan und Cyclohexan, insbesondere bevorzugt sind n-Pentan und n-Hexan. Es können auch Mischungen von Kohlenwasserstoffen wie z.B. Petrolether eingesetzt werden.

Vorteilhafte Cycloalkamonoene sind solche mit einer Ringgröße von 4 bis 12 Kohlenstoffatomen. Bevorzugte Cycloalkamonoene sind Cyclopenten, Cyclohexen, Cyclohepten, Cycloocten, Cyclononen, Cyclodecen und Cyclododecen. Insbesondere bevorzugt sind Cyclohepten und Cycloocten.

Besonders geeignete Cyclopolyene oder acyclische Polyene sind solche, welche aus den genannten Cycloalkamonoenen erhalten werden können. Die Cyclopolyene oder acyclische Polyene können beispielsweise als Nebenprodukte bei metathetischen Dimerisierungen, durch ringöffnende Metathesen oder Polymerisationen gebildet werden. Im allgemeinen weisen die Cyclopolyene und die acyclischen Polyene einen Polymerisierungsgrad von 3 bis 50, bevorzugt einen von 3 bis 20 auf. Unter dem Polymerisierungsgrad wird die Zahl der Monomereinheiten, gleich oder verschieden, verstanden, aus denen das Polyen aufgebaut ist.

Bevorzugte Cyclopolyene im Sinne der Erfindung sind Polymere bzw. Copolymere aus den genannten Cycloalkamonoenen, wobei die Cyclopolyene einen Polymerisierungsgrad von größer oder gleich drei, vorzugsweise 3 bis 50, insbesondere 3 bis 20, aufweisen. Bevorzugt werden Cyclopolyene des Cycloheptens, des Cyclooctens oder deren Copolymere verwendet.

Besonders bevorzugte Cyclopolyene sind Cyclopolyoctenylene der Formel 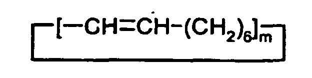 mit einem Polymerisierungsgrad m von mindestens 3, wobei m vorzugsweise im Bereich von 3 bis 50, insbesondere im Bereich von 3 bis 20 liegt.

Die Metathese-Lösungen können Cycloalkamonoene, Cyclopolyene, acyclische Polyene in beliebigen Zusammensetzungen und Mischungsverhältnissen enthalten. Bevorzugt sind Metathese-Lösungen, die Cycloalkamonoene enthalten. Werden Metathese-Lösungen verwendet, die nur Cycloalkamonoene als olefinische Verbindungen enthalten, so sind hier Cyclohepten, Cycloocten oder deren Mischungen bevorzugt. Weiterhin bevorzugt sind Mischungen aus Cycloalkamonoenen und Cyclopolyenen, wobei die Mischungen enthaltend Cyclohepten, Cycloocten oder deren Mischung und Cyclopolyheptenylen, Cyclopolyoctenylen, Copolymere aus Cyclohepten und Cycloocten oder deren Mischung besonders bevorzugt sind.

Werden Mischungen aus Cycloalkamonoenen und Cyclopolyenen eingesetzt, liegt das bevorzugte Gewichtsverhältnis im Bereich von 0,1 bis 2 zu 1, besonders bevorzugt im Verhältnis 0,2 bis 1 zu 1.

Ganz besonders bevorzugt ist eine Mischung aus Cycloocten und Cyclopolyoctenylen, wobei hier ein Verhältnis von Cycloocten zu Cyclopolyoctenylenen im Bereich 0,25 bis 0,5 zu 1 besonders bevorzugt ist.

Werden Cycloalkamonoene oder Mischungen enthaltend Cycloalkamonoene in die Metathesereaktion eingesetzt, so kann vorteilhafterweise ein Umsatz, bezogen auf den Gehalt an Cycloalkamonoenen, im Bereich von 40 bis 99 %, bevorzugt im Bereich 50 bis 95 %, insbesondere im Bereich 60 bis 85 %, eingestellt werden.

Die Metathese-Lösung kann auch geringe Anteilen an Cycloalkadienen, insbesondere den zu bildenden Cycloalkadienen, d.h. Produkt-Cycloalkadienen, enthalten. Diese können in geringen Mengen in den Cycloalkamonoenen, Cyclopolyenen oder den acyclischen Polyenen enthalten sein und beispielsweise destillativ bedingt sein.

Bevorzugte Cycloalkadiene, die nach dem erfindungsgemäßen Verfahren hergestellt werden können, sind bevorzugt solche mit 12 bis 18 Kohlenstoffatomen. Insbesondere bevorzugte Cycloalkadiene sind 1,8-Cyclotetradecadien, 1,8-Cyclopentadecadien und 1,9-Cyclohexadecadien. Ganz besonders bevorzugt ist 1,9-Cyclohexadecadien.

Die Metathesereaktion kann bei Temperaturen im Bereich von 0 bis 100°C durchgeführt werden, bevorzugt ist eine Temperatur im Bereich 25 bis 80°C, besonders bevorzugt eine im Bereich von 35 bis 60°C.

Beim Einsatz von Lösungsmitteln, deren Siedepunkt unterhalb der Reaktionstemperatur liegt, kann auch unter Druck gearbeitet werden. Im allgemeinen kann die Metathesereaktion bei einem Druck im Bereich von 1 bis 10 bar abs. durchgeführt werden.

Nach der Verwendung bei der Metathesereaktion kann der Trägerkatalysator regeneriert und erneut für die Metathesereaktion eingesetzt werden. Wie beispielsweise in EP-B1- 991 467 beschrieben, kann der Trägerkatalysator aus dem Metathesereaktor entfernt werden, mit einem Metathese-inerten Lösungsmittel gewaschen und anschließend getrocknet werden. Die thermische Behandlung des Trägerkatalysators bei der Regenerierung erfolgt in einem Temperaturbereich von 200 bis 600°C, wobei eine Maximaltemperatur von etwa 600°C einzuhalten ist. Die thermische Behandlung wird in sauerstoffhaltiger Atmosphäre durchgeführt, wie beispielsweise Luft, der gegebenenfalls zusätzlich Inertgase wie Stickstoff oder Argon beigemischt werden.

### Beispiele

Die folgenden Beispiele erläutern die Erfindung:

### Beispiel 1

Die Trägermaterialien aus γ-Aluminiumoxid wurden kommerziell erhalten (z.B. Fa. Condea, Fa. KataLeuna). Im Bedarfsfalle wurden einzelne Partikelfraktionen über Siebvorgänge erhalten.

Das γ-Aluminiumoxid (240 g) wurde als jeweiliger Formkörper mit einer wässrigen Lösung von Ammoniumperrhenat (16,5 g in 240 ml destilliertem Wasser) getränkt und getrocknet. Nach einer zweistündigen Behandlung bei 500 bis 580°C im Luftstrom wurde der Katalysator weitere 2 Stunden im Stickstoffstrom bei derselben Temperatur gehalten und anschließend auf Raumtemperatur abgekühlt. Die physikalischen Charakteristika sind in Tabelle 2 wiedergegeben.

**Tabelle 2:**

| Re₂O₇-haltige Trägerkatalysatoren | | | | | |
|---|---|---|---|---|---|
| | | Kat. 1 | Kat. 2 | Kat. 3 | Kat. 4 |
| Form | | Zylinder | Kugel | Kugel | Zylinder |
| Durchmesser Ø | mm | 1,7 | 1,5 | 1,0 | 0,8 |
| Länge | mm | 10 | - | - | 8 |
| spez. Oberfläche | mm²/mm³ | 2,55 | 4,00 | 6,00 | 5,25 |
| BET-Oberfläche | m²/g | 208 | 216 | 163 | 199 |

Der Re₂O₇-Gehalt lag bei allen in Tabelle 2 aufgeführten Trägerkatalysatoren bei 3,6 bis 3,7 Gew.%, der Gehalt an γ-Al₂O₃ bei 95,8 bis 96,0 Gew.%.

Die Katalysatoren aus Tabelle 2 wurden unter den in Beispiel 2 beschriebenen Bedingungen untersucht und mit dem kommerziell verfügbaren Kat. 1 (Extrudat in Zylinderform, Trägermaterial γ-Al₂O₃-CK-300 der Firma Akzo) verglichen (siehe Fig. 1).

### Beispiel 2

In einen vertikal aufgebauten Rohrreaktor (Höhe: 50 cm, Durchmesser: 1,5 cm) wurden jeweils 50 g der einzelnen in Tabelle 2 aufgeführten Trägerkatalysatoren unter Schutzgasatmosphäre (Argon) eingefüllt. Über eine Pumpe wurde eine Lösung, enthaltend 2,5 Gew.% Zinntetramethyl (bezogen auf das Trägerkatalysatorgewicht) 3 Stunden lang bei 30°C in n-Hexan von unten nach oben im Kreis über das Festbett des Trägerkatalysators geführt. Das Trägerkatalysatorbett wurde danach bei Normaldruck und bei 45°C kontinuierlich von einer Lösung enthaltend 2,4 g Cycloocten und 0,5 Gew.% Zinntetramethyl (bezogen auf das Trägerkatalysatorgewicht) pro Liter n-Hexan von unten angeströmt.

Die pro Zeiteinheit über das Trägerkatalysatorbett geführte Menge an Metathese-Lösung, d.h. die Raum-Zeit-Geschwindigkeit, wurde über die Pumpenleistung variiert.

Die Selektivität an 1,9-Cyclohexadecadien lag über den gesamten Umsatzbereich bei 36 bis 38 %. Die Selektivität bezogen auf 1,9-Cyclohexadecadien und Cyclopolyoctenylenen lag bei 99 %.

In Fig. 1 ist der Cycloocten-bezogene Umsatz (in Prozent, x-Achse) in Abhängigkeit von der Raum-Zeit-Geschwindigkeit (in ml / gh, y-Achse) in einer Metathesereaktion mit den erfindungsgemäßen Trägerkatalysatoren (Kat. 2 bis 4) im Vergleich zu dem kommerziell erhältlichen Trägerkatalysator Kat. 1 graphisch dargestellt. Die erfindungsgemäßen Trägerkatalysatoren zeigen eine deutlich höhere Aktivität.

### Beispiel 3

Das γ-Aluminiumoxid (240 g) wurde in Form von 1,0 mm Kugeln (erhältlich von der Firma Condea) mit einer Lösung von Rheniumoxid (9 g in 120 g destilliertem Wasser) getränkt und anschließend getrocknet. Nach einer zweistündigen Behandlung bei 500 bis 580°C im Luftstrom wurde der Katalysator weitere 2 Stunden im Stickstoffstrom bei derselben Temperatur gehalten und anschließend auf Raumtemperatur abgekühlt. Nach der Tränkung mit einer wässrigen Cäsiumnitrat-Lösung (1,8 g Cäsiumnitrat in 125 ml destilliertem Wasser), wurde der Katalysator zwei Stunden bei 120°C getrocknet, gefolgt von einer zweistündigen Behandlung bei 500°C im Luftstrom und Abkühlung im Stickstoffstrom. Auf diese Weise wurde ein Trägerkatalysator in 1,0 mm Kugelform hergestellt, der 3,6 Gew.% Re₂O₇ und 0,5 Gew.% Cäsium enthielt.

## Patentansprüche

1. Verfahren zur Herstellung von Cycloalkadienen aus Cycloalkamonoenen, Cyclopolyenen, acyclischen Polyenen oder deren Gemische in Flüssigphase durch Metathesereaktion in Gegenwart eines Trägerkatalysator- Formkörpers auf Basis Re₂O₇/γ-Al₂O₃, **dadurch gekennzeichnet, dass** die berechnete spezifische äußere Oberfläche des Trägerkatalysator-Formkörpers größer oder gleich 3,5 mm²/mm³ ist.

2. Verfahren nach Anspruch 1, **dadurch gekennzeichnet, dass** die berechnete spezifische Oberfläche des Trägerkatalysator-Formkörpers größer oder gleich 4,0 mm²/mm³ ist.

3. Verfahren nach den Ansprüchen 1 oder 2, **dadurch gekennzeichnet, dass** als Formkörper Kugeln, Zylinder oder Wirbelstränge eingesetzt werden.

4. Verfahren nach mindestens einem der Ansprüche 1 bis 3, **dadurch gekennzeichnet, dass** der Trägerkatalysator einen Gehalt an Re₂O₇ im Bereich von 1 bis 12 Gew.% aufweist.

## Claims

1. Process for the preparation of cycloalkadienes from cycloalkamonoenes, cyclopolyenes, acyclic polyenes or mixtures thereof in the liquid phase, by metathesis reaction in the presence of a shaped supported catalyst body based on Re₂O₇/γ-Al₂O₃, **characterised in that** the calculated specific external surface area of the shaped supported catalyst body is greater than or equal to 3.5 mm²/mm³.

2. Process according to claim 1, **characterised in that** the calculated specific surface area of the shaped supported catalyst body is greater than or equal to 4.0 mm²/mm³.

3. Process according to claim 1 or 2, **characterised in that** spheres, cylinders or swirl strands are used as the shaped body.

4. Process according to at least one of claims 1 to 3, **characterised in that** the supported catalyst has a Re₂O₇ content in the range from 1 to 12 wt.%.

## Revendications

1. Procédé pour la préparation de cycloalcadiènes à partir de cycloalcamonoènes, cyclopolyènes, polyènes acycliques ou mélanges de ceux-ci en phase liquide par réaction de métathèse en présence d'un corps moulé de catalyseur sur support à base de Re₂O₇/γ-Al₂O₃, **caractérisé en ce que** la surface externe spécifique calculée du corps moulé de catalyseur sur support est supérieure ou égale à 3,5 mm²/mm³.

2. Procédé selon la revendication 1, **caractérisé en ce que** la surface spécifique calculée du corps moulé de catalyseur sur support est supérieure ou égale à 4,0 mm²/mm³.

3. Procédé selon les revendications 1 ou 2, **caractérisé en ce que** l'on utilise comme corps moulés des sphères, des cylindres ou des barres en forme de vertèbre.

4. Procédé selon au moins l'une quelconque des revendications 1 à 3, **caractérisé en ce que** le catalyseur sur support présente une teneur en Re₂O₇ dans le domaine de 1 à 12 % en poids.
